# FASCICULE DE BREVET EUROPEEN

(11) **EP 1 335 698 B1**
(45) Date de publication et mention de la délivrance du brevet: **17.01.2007**
(21) Numéro de dépôt: 01993451.2
(22) Date de dépôt: 06.11.2001
(51) Int. Cl.: A61Q 5/04, A61Q 5/08, A61K 8/87

(54) **COMPOSITION POUR LA DECOLORATION OU LA DEFORMATION PERMANENTE DES FIBRES KERATINIQUES COMPRENANT UN POLYURETHANE ASSOCIATIF CATIONIQUE**
ZUSAMMENSETZUNG ZUR ENTFÄRBUNG ODER ZUR DAUERWELLUNG KERATINISCHER FASERN ENTHALTEND EIN ASSOZIATIVES KATIONISCHES POLYURETHAN
COMPOSITION FOR BLEACHING OR PERMANENT WAVING OF KERATINOUS FIBRES COMPRISING A CATIONIC ASSOCIATIVE POLYURETHANE

(30) Priorité: 08.11.2000 FR 0014321
(43) Date de publication de la demande: 20.08.2003
(73) Titulaire: L'ORÉAL, 75008 Paris (FR)
(72) Inventeur: LEGRAND, Frédéric, F-92400 Courbevoie (FR); DE LA METTRIE, Roland, F-78110 Le Vesinet (FR)
(74) Mandataire: Wattremez, Catherine
(86) Numéro de dépôt international: PCT/FR2001/003430
(87) Numéro de publication internationale: WO 2002/038118

(56) Documents cités:
- FR-A- 2 773 991
- US-A- 5 650 159

## Description

La présente invention concerne une composition pour la décoloration ou la déformation permanente des fibres kératiniques, en particulier des fibres kératiniques humaines et plus particulièrement des cheveux, comprenant au moins un agent réducteur et au moins un polyuréthane associatif cationique.

Il est connu de décolorer les fibres kératiniques et en particulier les cheveux humains, avec des compositions de décoloration contenant un ou plusieurs agents oxydants. Parmi les agents oxydants classiquement utilisés, on peut citer le peroxyde d'hydrogène ou des composés susceptibles de produire le peroxyde d'hydrogène par hydrolyse, tels que le peroxyde d'urée ou les persels comme les perborates, les percarbonates et les persulfates, le peroxyde d'hydrogène et les persulfates étant particulièrement préférés.

Mais il est également connu de décolorer les fibres kératiniques humaines telles que les cheveux et en particulier les cheveux teints artificiellement avec des colorants exogènes, à l'aide d'agents réducteurs tels que l'acide ascorbique ou les thiols comme la cystéine. Il est connu aussi de procéder à la déformation permanente des cheveux en appliquant sur ceux-ci des compositions contenant un ou plusieurs réducteurs, les cheveux ayant été, de préférence, préalablement mis sous tension, en particulier à l'aide de dispositifs mécaniques tels que des bigoudis, les cheveux ainsi réduits étant alors réoxydés dans la forme souhaitée, le plus souvent après rinçage, par l'intermédiaire de l'oxygène de l'air, mais plus généralement via un oxydant qui est de préférence choisi parmi l'eau oxygénée ou les bromates alcalins.
Les réducteurs préférentiellement utilisés dans le cadre de la déformation permanente des cheveux sont les thiols tels que l'acide thioglycolique, ses sels et ses esters, l'acide thiolactique et ses sels, la cystéine ou la cystéamine, et les sulfites.

Les compositions destinées à la décoloration des cheveux à l'aide d'agents réducteurs se présentent principalement sous forme de compositions prêtes à l'emploi constituées de produits anhydres (poudres ou crèmes) contenant le ou les agents réducteurs que l'on mélange au moment de l'emploi avec une composition aqueuse contenant éventuellement un agent de pH. Les compositions de décoloration se présentent également sous forme de compositions prêtes à l'emploi aqueuses contenant le ou les agents réducteurs au pH approprié.

Les compositions réductrices pour la déformation permanente des cheveux se présentent généralement sous forme de compositions aqueuses prêtes à l'emploi ou sous forme de compositions anhydres pulvérulents ou liquides que l'on mélange au moment de l'emploi avec une composition aqueuse au pH approprié.

Pour localiser le produit de décoloration ou de déformation permanente à l'application sur les cheveux, afin qu'il ne coule pas sur le visage ou en' dehors des zones que l'on se propose de traiter, on a jusqu'ici eu recours à l'emploi d'épaississants traditionnels tels que l'acide polyacrylique réticulé, les hydroxyéthylcelluloses, certains polyuréthanes, les cires, et en outre, dans le cas des compositions de décoloration, à des mélanges d'agents tensio-actifs non-ioniques de HLB (Hydrophilic Lipophilic Balance), qui, convenablement choisis, engendrent l'effet gélifiant quand on les dilue au moyen d'eau et/ou d'agents tensio-actifs.

Le document EP-A-1 036 558 décrit une composition cosmétique anhydre pour la décoloration des fibres kératiniques contenant des épaississants qui pourraient être choisi généralement entre les polyuréthanes.

Cependant, la demanderesse a constaté que les systèmes épaississants mentionnés ci-dessus ne permettaient pas d'obtenir des décolorations ou des déformations permanentes suffisamment puissantes et homogènes.
Par ailleurs, elle a également constaté que les compositions de décoloration ou de déformation permanente des cheveux, prêtes à l'emploi, contenant le ou les agents réducteurs, et en outre les systèmes épaississants de l'art antérieur ne permettaient pas une application suffisamment précise sans coulures ni chutes de viscosité dans le temps.

Or, après d'importantes recherches menées sur la question, la Demanderesse vient maintenant de découvrir qu'il est possible d'obtenir des compositions de décoloration ou de déformation permanente des cheveux contenant au moins un agent réducteur, prêtes à l'emploi, qui ne coulent pas et restent donc bien localisées au point d'application, et qui permettent aussi d'obtenir des décolorations ou des déformations permanentes puissantes et homogènes, si on introduit dans la composition une quantité efficace d' un polymère polyuréthane associatif cationique

Ces découvertes sont à la base de la présente invention.

La présente invention a ainsi pour objet une composition prête à l'emploi pour la décoloration ou la déformation permanente des fibres kératiniques, en particulier des fibres kératiniques humaines et plus particulièrement les cheveux, comprenant, dans un milieu approprié pour la décoloration ou la déformation permanente, au moins un agent réducteur, qui est caractérisée par le fait qu'elle comprend en outre au moins un polyuréthane associatif cationique.

Par "composition prête à l'emploi" on entend, au sens de l'invention, la composition destinée à être appliquée telle quelle sur les fibres kératiniques, c'est à dire qu'elle peut être stockée telle quelle avant utilisation ou résulter du mélange extemporané de deux ou plusieurs compositions.

Lorsque la composition prête à l'emploi selon l'invention résulte du mélange extemporané de plusieurs compositions, le polyuréthane associatif cationique peut être présent dans une ou plusieurs ou dans la totalité des compositions mélangées.
De la sorte, le polyuréthane associatif cationique peut être présent dans une composition anhydre sous forme de poudre de préférence pulvérulente ou de crème et/ou dans une ou plusieurs compositions aqueuses.

De préférence selon l'invention, le ou les polyuréthanes associatifs cationiques sont présents dans au moins une composition aqueuse que l'on mélange au moment de l'emploi avec une composition soit aqueuse soit anhydre sous forme de poudre ou de crème et contenant au moins un agent réducteur.
Une autre forme préférée de l'invention est une composition unique contenant le ou les agents réducteurs et le ou les polyuréthanes associatifs cationiques.
Un autre objet visé par la présente invention est une composition anhydre comprenant au moins un agent réducteur et au moins un polyuréthane associatif cationique, ladite composition étant destinée à être diluée avant application sur les fibres.

L'invention vise également un procédé de décoloration ou un procédé de déformation permanente des fibres kératiniques, en particulier des fibres kératiniques humaines et plus particulièrement les cheveux utilisant la composition de décoloration ou de déformation permanente prête à l'emploi telle que décrite selon l'invention, l'application de ladite composition pouvant être suivie, dans le cas de la déformation permanente, par l'application, éventuellement après rinçage, d'une composition oxydante.

L'invention vise aussi des dispositifs ou "Kits" d'emballage pour la décoloration ou la déformation permanente des fibres kératiniques humaines et plus particulièrement des cheveux, comprenant une telle composition prête à l'emploi.

Ainsi, un dispositif à deux compartiments comprend un premier compartiment comprenant au moins une poudre ou une crème anhydre ou une composition aqueuse, et le deuxième compartiment une composition aqueuse, l'un au moins des deux compartiments comprenant au moins un agent réducteur et l'un au moins des deux compartiments comprenant au moins un polyuréthane associatif cationique.

Mais d'autres caractéristiques, aspects, objets et avantages de l'invention apparaîtront encore plus clairement à la lecture de la description et des exemples qui suivent.

Les polymères associatifs sont des polymères dont les molécules sont capables, dans le milieu de formulation, de s'associer entre elles ou avec des molécules d'autres composés.
Un cas particulier de polymères associatifs sont des polymères amphiphiles, c'est-à-dire des polymères comportant une ou plusieurs parties hydrophiles qui les rendent solubles dans l'eau et une ou plusieurs zones hydrophobes (comprenant au moins une chaîne grasse) par lesquelles les polymères interagissent et se rassemblent entre eux ou avec d'autres molécules.

### Polyuréthanes associatifs cationiques

Les polyuréthanes associatifs cationiques selon la présente invention sont plus particulièrement choisis parmi les polyuréthanes amphiphiles associatifs cationiques, hydrosolubles ou hydrodispersibles.
Le terme "hydrosoluble" ou "soluble dans l'eau" concernant les polyuréthanes associatifs de la présente invention signifie que ces polymères ont une solubilité dans l'eau à température ambiante au moins égale à 1 % en poids, c'est-à-dire que jusqu'à cette concentration, aucun précipité ne peut être détecté à l'oeil nu et la solution est parfaitement limpide et homogène.
On entend par polyuréthanes "hydrodispersibles" ou "dispersibles dans l'eau" des polymères qui, lorsqu'on les met en suspension dans l'eau, forment spontanément des globules ayant une taille moyenne, mesurée par diffusion de la lumière sur un appareil de type Coulter, comprise entre 5 nm et 600 nm, et en particulier entre 5 nm et 500 nm.

La famille de polyuréthanes associatifs cationiques selon l'invention a été décrite par la demanderesse dans la demande de brevet français N°-0009609; elle peut être représentée par la formule générale (la) suivante :

R-X-(P)ₙ-[L-(Y)ₘ]ᵣ-L'-(P')ₚ-X'-R' (la)

dans laquelle :
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ;
la molécule contenant au moins une fonction amine protonée ou quaternisée et au moins un groupement hydrophobe.

Dans un mode de réalisation préféré des polyuréthanes de la présente invention, les seuls groupements hydrophobes sont les groupes R et R' aux extrémités de chaîne.

Une famille préférée de polyuréthanes associatifs cationiques est celle correspondant à la formule (la) décrite ci-dessus et dans laquelle :
R et R' représentent tous les deux indépendamment un groupement hydrophobe,
X, X' représentent chacun un groupe L",
n et p valent entre 1 et 1000 et
L, L', L", P, P', Y et m ont la signification indiquée ci-dessus.

Une autre famille préférée de polyuréthanes associatifs cationiques est celle correspondant à la formule (la) ci-dessus dans laquelle :
R et R' représentent tous les deux indépendamment un groupement hydrophobe, X,
X' représentent chacun un groupe L", n et p valent 0, et L, L', L", Y et m ont la signification indiquée ci-dessus.
Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Encore une autre famille préférée de polyuréthanes associatifs cationiques est celle correspondant à la formule (la) ci-dessus dans laquelle :
R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée ci-dessus.

La masse moléculaire moyenne en nombre des polyuréthanes associatifs cationiques est comprise de préférence entre 400 et 500 000, en particulier entre 1000 et 400 000 et idéalement entre 1000 et 300 000.
Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.
Préférentiellement, le groupement hydrocarboné provient d'un composé monofonctionnel.
A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Lorsque X et/ou X' désignent un groupement comportant une amine tertiaire ou quaternaire, X et/ou X' peuvent représenter l'une des formules suivantes : 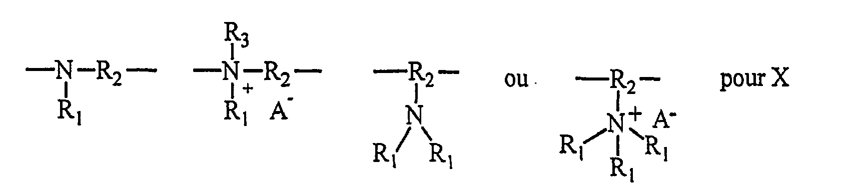 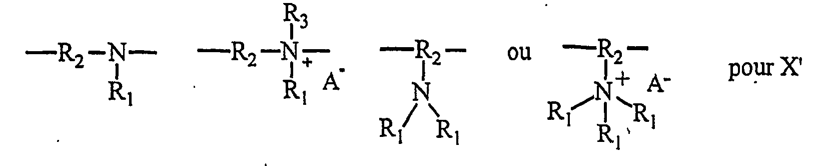 dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A⁻ est un contre-ion physiologiquement acceptable.

Les groupements L, L' et L" représentent un groupe de formule : 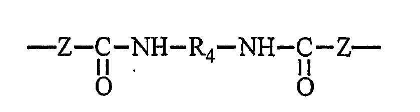 dans laquelle :
Z représente -O-, -S- ou -NH- ; et
R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 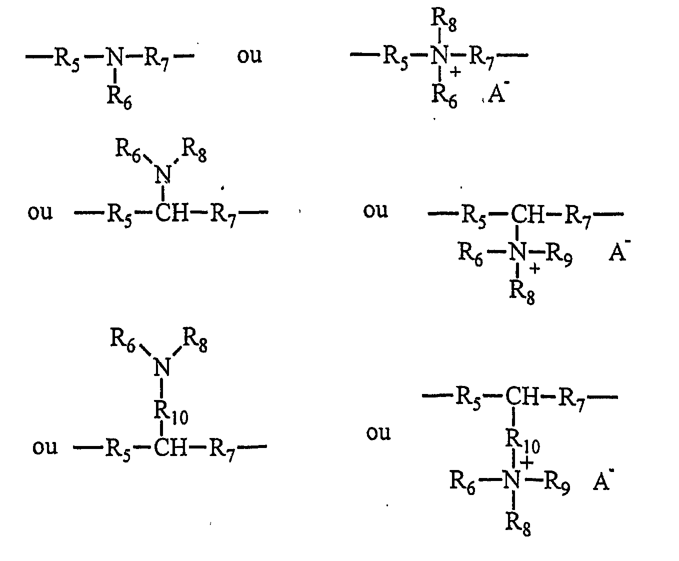 dans lesquelles :
R₅ et R₇ ont les mêmes significations que R₂ défini précédemment;
R₆, R₈ et R₉ ont les mêmes significations que R₁ et R₃ définis précédemment ;
R₁₀ représente un groupe alkylène, linéaire ou ramifié, éventuellement insaturé et pouvant contenir un ou plusieurs hétéroatomes choisis parmi N, O, S et P,
et A⁻ est un contre-ion physiologiquement acceptable.

En ce qui concerne la signification de Y, on entend par groupement hydrophile, un groupement hydrosoluble polymérique ou non.
A titre d'exemple, on peut citer, lorsqu'il ne s'agit pas de polymères, l'éthylèneglycol, le diéthylèneglycol et le propylèneglycol.
Lorsqu'il s'agit, conformément à un mode de réalisation préféré de l'invention, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs cationiques de formule (la) selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" de la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (la) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.
Comme indiqué précédemment, ce composé peut comporter plus d'un motif à fonction amine. Il s'agit alors d'un polymère portant une répétition du motif à fonction amine.

Ce type de composés peut être représenté par l'une des formules suivantes :

HZ-(P)ₙ-ZH,

ou

HZ-(P')p-ZH

dans lesquelles Z, P, P', n et p sont tels que définis plus haut.

A titre d'exemple de composé à fonction amine, on peut citer la N-méthyldiéthanolamine, la N-tert-butyl-diéthanolamine, la N-suifoéthyldiéthanolamine.

Le deuxième composé entrant dans la préparation du polyuréthane de formule (la) est un diisocyanate correspondant à la formule :

O=C=N-R₄-N=C=O

dans laquelle R₄ est défini plus haut.

A titre d'exemple, on peut citer le méthylènediphényl-diisocyanate, le méthylènecyclohexanediisocyanate, l'isophorone-diisocyanate, le toluènediisocyanate, le naphtalènediisocyanate, le butanediisocyanate, l'hexanediisocyanate.

Un troisième composé entrant dans la préparation du polyuréthane de formule (la) est un composé hydrophobe destiné à former les groupes hydrophobes terminaux du polymère de formule (la).
Ce composé est constitué d'un groupe hydrophobe et d'une fonction à hydrogène labile, par exemple une fonction hydroxyle, amine primaire ou secondaire, ou thiol. A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné - hydroxyle.
Le groupe hydrophobe du polyuréthane de formule (la) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.
A titre d'exemple, on peut citer, lorsqu'il ne s'agit pas de polymères, l'éthylèneglycol, le diéthylèneglycol et le propylèneglycol.
Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (la) est facultatif. En effet, les motifs à fonction amine quaternaire ou protonée peuvent suffire à apporter la solubilité ou l'hydrodispersibilité nécessaire pour ce type de polymère dans une solution aqueuse.
Bien que la présence d'un groupe Y hydrophile soit facultative, on préfère cependant des polyuréthanes associatifs cationiques comportant un tel groupe.
Lesdits polyuréthanes associatifs cationiques sont hydrosolubles ou hydrodispersibles.

Le ou les polyuréthanes associatifs cationiques sont utilisés de préférence en une quantité pouvant varier d'environ 0,01 à 10% en poids du poids total de la composition de teinture prête à l'emploi. Plus préférentiellement, cette quantité varie d'environ 0,1 à 5% en poids.

Les agents réducteurs utilisables selon l'invention sont choisis de préférence parmi les thiols tels que la cystéine, l'acide thioglycolique, l'acide thiolactique, leurs sels et leurs esters, la cystéamine et ses sels ou les sulfites.
Dans le cas des compositions destinées à la décoloration, on peut aussi utiliser l'acide ascorbique, ses sels et ses esters, l'acide érythorbique, ses sels et ses esters, les sulfinates comme l'hydroxyméthanesulfinate de sodium.

Ces réducteurs sont utilisés dans lesdites compositions prêtes à l'emploi dans des concentrations comprises entre 0,1 et 30% environ, de préférence entre 0,5 et 20% environ en poids, par apport au poids total de la composition.

Plus particulièrement, les compositions selon l'invention peuvent en outre comprendre, au moins un polymère substantif amphotère ou cationique différent des polyuréthanes associatifs cationiques de l'invention.

### Polymères cationiques

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.
Ce sont des produits connus. Ils sont notamment décrits dans les brevets français n° 2 505 348 ou 2 542 997. Parmi lesdits polymères, on peut citer :
**(1)** Les homopolymères ou copolymères dérivés d'esters ou d'amides acryliques ou méthacryliques et comportant au moins un des motifs de formules (I), (II), (III) ou (IV) suivantes: 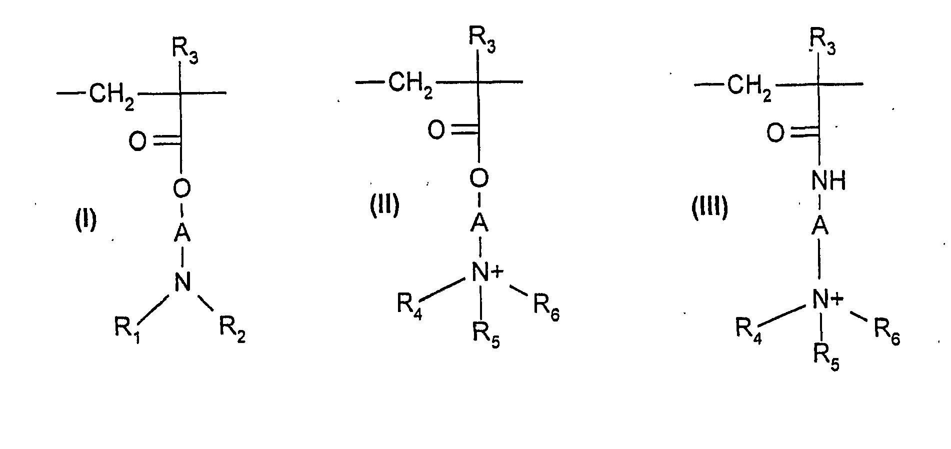 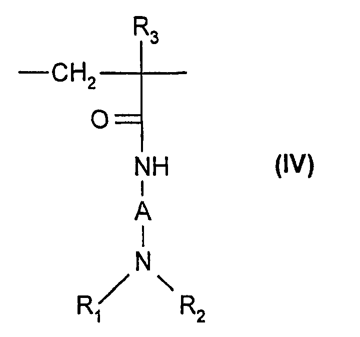 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.

   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques. Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP,
   - et les copolymères vinylpyrrolidone / méthacrylamide de diméthylaminopropyle quaternisés tel que le produit vendu sous la dénomination "GAFQUAT HS 100" par la société ISP.
**(2)** Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
**(3)** Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
**(4)** Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
**(6)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
**(7)** Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
**(8)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
**(9)** Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 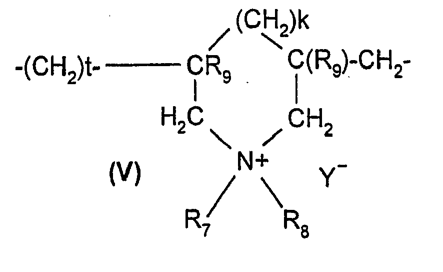 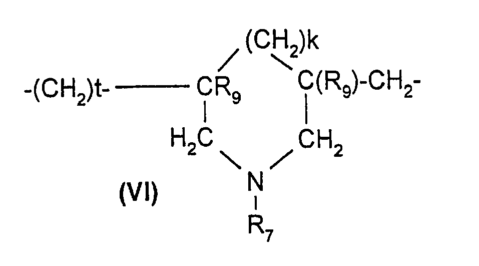 formules dans lesquelles k et t sont égaux à 0 ou 1, la somme k + t étant égale à 1 ; Rg désigne un atome d'hydrogène ou un radical méthyle ; R₇ et R₈, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
**(10)** Le polymère de diammonium quaternaire contenant des motifs récurrents répondant à la formule : 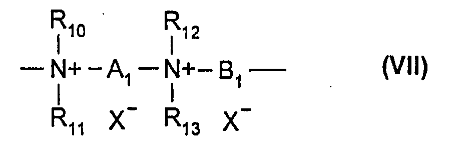 formule (VII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH-R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement - (CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂-;
      d) un groupement uréylène de formule : -NH-CO-NH-.
      De préférence, X⁻ est un anion tel que le chlorure ou le bromure.
      Ces polymères ont une masse moléculaire moyenne en nombre généralement comprise entre 1000 et 100000.
      Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

   On peut utiliser plus particulièrement les polymères qui sont constitués de motifs récurrents répondant à la formule (VIII) suivante: 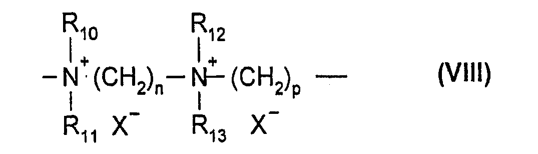 dans laquelle R₁₀, R₁₁, R₁₂ et R_{13,} identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
**(11)** Les polymères de polyammonium quaternaire constitués de motifs récurrents de formule (IX) : 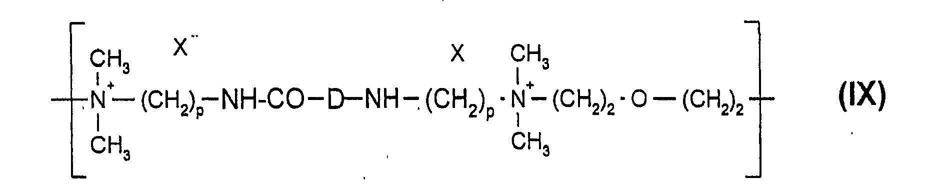 dans laquelle p désigne un nombre entier variant de 1 à 6 environ, D peut être nul ou peut représenter un groupement -(CH₂)ᵣ -CO- dans lequel r désigne un nombre égal à 4 ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol A 15", "Mirapol AD1", "Mirapol AZ1" et "Mirapol 175" vendus par la société Miranol.
**(12)** Les polymères quaternaires de vinylpyrrolidone et de vinylimidazole tels que par exemple les produits commercialisés sous les dénominations Luviquat FC 905, FC 550 et FC 370 par la société B.A.S.F.
**(13)** Les polyamines comme le Polyquart H vendu par HENKEL, référencé sous le nom de " POLYETHYLENEGLYCOL (15) TALLOW POLYAMINE " dans le dictionnaire CTFA.
**(14)** Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE^{®} SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes : 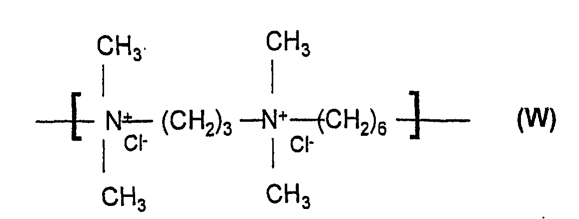 et notamment ceux dont le poids moléculaire, déterminé par chromatographie par perméation de gel, est compris entre 9500 et 9900; 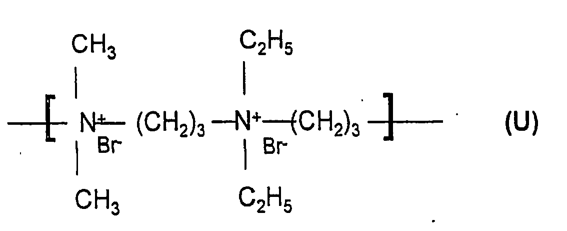 et notamment ceux dont le poids moléculaire, déterminé par chromatographie par perméation de gel, est d'environ 1200.

La concentration en polymère cationique dans la composition selon la présente invention peut varier de 0,01 à 10% en poids par rapport au poids total de la composition, de préférence de 0,05 à 5% et plus préférentiellement encore de 0,1 à 3%.

### Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl aminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) Les polyaminoamides réticulés et alcoylés partiellement ou totalement dérivant de polyaminoamides de formule générale :

   ⁅CO-R₁₉-CO-Z⁆ (X)

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 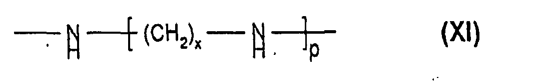 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine : 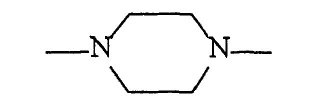
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.

   Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 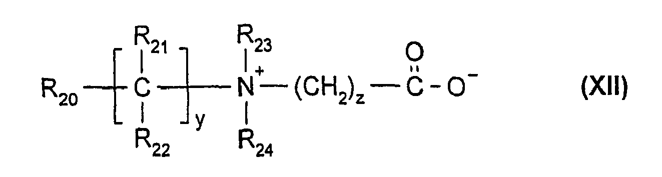 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.
(5) les polymères dérivés du chitosane décrits notamment dans les brevets français N°-2137684 ou US-3879376, comportant des motifs monomères répondant aux formules (XIII), (XIV), (XV) suivantes réunies dans leur chaîne: 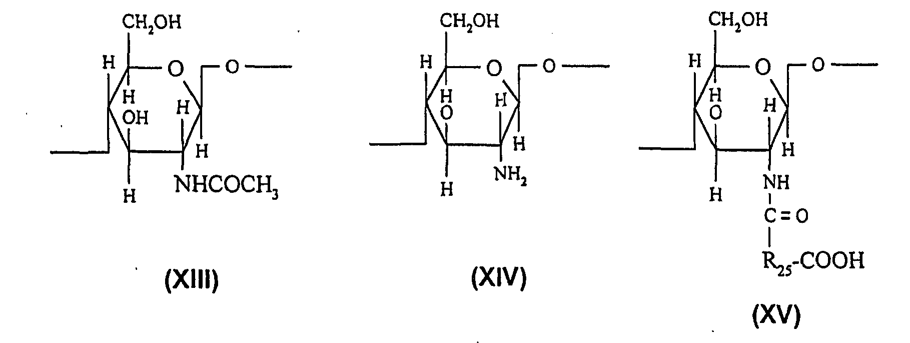 le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), R₂₅ représente un radical de formule : 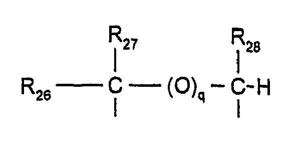 dans laquelle q désigne zéro ou 1 ;
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (XI) tels que ceux décrits par exemple dans le brevet français 1 400 366 : 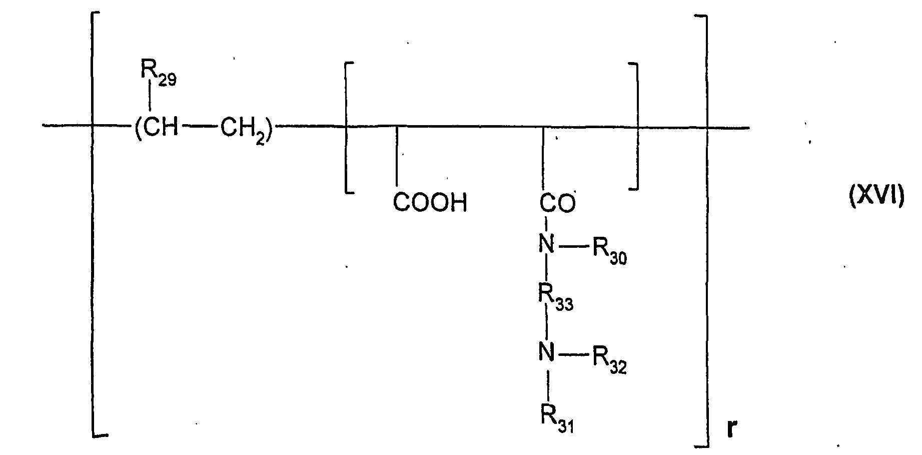 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁ désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂- , -CH₂-CH₂-CH₂-, -CH₂-CH(CH₃)- , R₃₁ ayant les significations mentionnées ci-dessus,
   ainsi que les homologues supérieurs de ces radicaux et contenant jusqu'à 6 atomes de carbone,
   r est tel que le poids moléculaire est compris entre 500 et 6000000 et de préférence entre 1000 et 1000000.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (XVII)

      où D désigne un radical 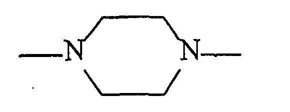 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- (XVIII)
   où D désigne un radical 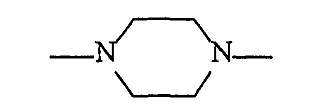 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères amphotères peuvent représenter de 0,01 % à 10 % en poids, de préférence de 0,05 % à 5 % en poids, et encore plus préférentiellement de 0,1 % à 3 % en poids, du poids total de la composition.

Les compositions de l'invention comprennent de préférence un ou plusieurs tensioactifs. Le ou les tensioactifs peuvent être indifféremment choisis, seuls ou en mélanges, au sein des tensioactifs anioniques, amphotères, non ioniques, zwittérioniques et cationiques.

Les tensioactifs convenant à la mise en oeuvre de la présente invention sont notamment les suivants :

### (i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO⁻)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂' désigne un radical alkyle d'un acide Rg -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Coco-amphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL^{®} C2M concentré par la société RHODIA CHIMIE.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent varier de 0,01 à 40% et de préférence de 0,5 à 30% du poids total de la composition.

Les compositions selon l'invention peuvent également comprendre d'autres agents d'ajustement de la rhéologie tels que les épaississants cellulosiques (hydroxyéthycellulose, hydroxypropylcellulose, carboxyméthylcellulose..), la gomme de guar et ses dérivés( hydroxypropylguar..), les gommes d'origine microbienne (gomme de xanthane, gomme de scléroglucane..), les épaississants synthétiques tels que les homopolymères réticulés d'acide acrylique ou d'acide acrylamidopropanesulfonique et les polymères associatifs ioniques ou non ioniques tels que les polymères commercialisés sous les appellations PEMULEN TR1 ou TR2 par la société GOODRICH, SALCARE SC90 par la société ALLIED COLLOIDS, ACULYN 22, 28, 33, 44, ou 46 par la société ROHM & HAAS et ELFACOS T210 et T212 par la société AKZO.

Ces épaississants d'appoint peuvent représenter de 0,05 à 10% en poids du poids total de la composition.

Le pH de la composition prête à l'emploi est généralement compris entre environ 1,5 et 12.
Plus préférentiellement, le pH des compositions de l'invention prêtes à l'emploi destinées à la décoloration est compris entre environ 1,5 et 10, et encore plus préférentiellement entre environ 1,5 et 7.
Plus préférentiellement, le pH des compositions de l'invention prêtes à l'emploi destinées à la déformation permanente est compris entre environ 6 et 12 et encore plus préférentiellement entre environ 7 et 11.

Ce pH peut être ajusté à la valeur désirée au moyen d'agents acidifiants ou alcalinisants bien connus de l'état de la technique en décoloration ou en déformation permanente des fibres kératiniques.

Parmi les agents alcalinisants on peut citer, à titre d'exemple, l'ammoniaque, les carbonates alcalins ou d'ammonium, les alcanolamines telles que les mono-, di- et triéthanolamines ainsi que leurs dérivés, les hydroxyalkylamines et les éthylènediamines oxyéthylénées et/ou oxypropylénées, les hydroxydes de sodium ou de potassium et les composés de formule (XIX) suivante : 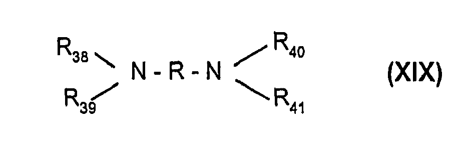 dans laquelle R est un reste propylène éventuellement substitué par un groupement hydroxyle ou un radical alkyle en C₁-C₄ ; R₃₈, R₃₉, R₄₀ et R₄₁, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Les agents alcalinisants ou acidifiants peuvent représenter environ 0,01 à 30% en poids du poids total de la composition de décoloration ou de déformation permanente.

Les compositions de l'invention peuvent également comprendre des agents séquestrants comme par exemple l'acide éthylènediamine tétraacétique (EDTA).

Lorsque les compositions comprenant l'agent réducteur et le polyuréthane associatif cationique sont sous forme anhydre ( poudre ou crème ), elles comprennent les agents principaux et additifs mentionnés ci-dessus sous forme de solides ou de liquides essentiellement anhydres.
Elles peuvent également comprendre des charges minérales ou organiques telles que la silice ou les argiles, des liants tels que la vinylpyrrolidone, les huiles ou les cires, les polyalkylèneglycols ou les dérivés de polyalkylèneglycols, des lubrifiants comme les stéarates de polyol ou les stéarates de métaux alcalins ou alcalino-terreux, ainsi que des agents colorants ou matifiants comme les oxydes de titane, chacun de ces additifs pouvant être présent à une concentration allant de 0 à 30% en poids du poids total de la composition.

Lorsque le milieu contenant l'agent réducteur est un milieu aqueux, il peut éventuellement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,5 et 20% et, de préférence, entre environ 2 et 10% en poids par rapport au poids total de la composition.

La composition de décoloration ou de déformation permanente selon l'invention peut encore comprendre une quantité efficace d'autres agents, par ailleurs antérieurement connus en décoloration ou en déformation permanente des fibres kératiniques, tels que divers adjuvants usuels comme des silicones volatiles ou non, cycliques ou linéaires ou ramifiées, organomodifiées (notamment par des groupements amines) ou non organomodifiées, des conservateurs, des céramides, des cires ou des huiles, végétales, minérales ou synthétiques, des acides et en particuliers des AHA, d'autres polymères associatifs que ceux de l'invention et en particulier des polyuréthanes polyéthers associatifs non ioniques, etc...

Bien entendu, l'homme de l'art veillera à choisir le ou les éventuels composés complémentaires mentionnés ci-avant, de manière telle que les propriétés avantageuses attachées intrinsèquement à la composition de décoloration ou de déformation permanente des fibres kératiniques selon l'invention ne soient pas, ou substantiellement pas, altérées par la ou les adjonctions envisagées.

De préférence, le procédé de décoloration selon l'invention consiste à appliquer la composition réductrice prête à l'emploi, sur les fibres kératiniques sèches ou humides, et à la laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

De préférence, le procédé de déformation permanente selon l'invention consiste à appliquer la composition réductrice prête à l'emploi sur les fibres kératiniques sèches ou humides, à la laisser agir pendant un temps de pause variant, de préférence, de 1 à 60 minutes environ, et plus préférentiellement de 10 à 45 minutes environ, à rincer éventuellement les fibres, puis à appliquer une composition oxydante et laisser agir pendant un temps de pause compris entre 1 et 20 minutes et de préférence entre 1 et 10 minutes, puis éventuellement à les laver au shampooing, puis à les rincer à nouveau, et à les sécher.
Des moyens mécaniques de mise sous tension des fibres kératiniques peuvent être utilisés avant, pendant ou après application de la composition réductrice et ôtés avant ou après le rinçage de la composition oxydante.

Des exemples concrets illustrant l'invention sont indiqués ci-après, sans pour autant présenter un caractère limitatif.

### EXEMPLE 1 :

On a préparé les compositions de décoloration aqueuses prêtes à l'emploi suivantes (exprimées en grammes):

| | A | B |
|---|---|---|
| Acide citrique | 7,4 | 7,4 |
| Citrate trisodique dihydraté | 1 | 1 |
| Hydroxyéthylcellulose | 1,5 | 1,5 |
| Acide 2-oxoglutarique | 0,8 | 0,8 |
| Ascorbate de sodium | 5,7 | 5,7 |
| L-cystéine | 2 | 2 |
| Polymère 1 | 0,3 MA* | |
| Polymère 2 | | 0,3 MA* |
| Sulfate de magnésium | 1 | 1 |
| Eau .qsp | 100 | 100 |

| | | |
|---|---|---|
| MA*= Matière Active | | |

Le polymère 1 est le polymère suivant :
C₁₈H₃₇-O-CONHR₄NHCO-O-(CH₂)₂-N⁺(CH₃)(CH₃)-CH₂)₂-O-CONHR₂NHCO-O(POE)O-CONHR₂NHCO-O-(CH₂)₂₋N⁺(CH₃)(CH₃)-(CH₂)₂-O-CONHR₄NHCO-OC₁₈H₃₇
avec :
R₄ = méthylènedicyclohexyle
contre ion : CH₃SO₄⁻

Il est synthétisé à partir des réactifs suivants :

| | |
|---|---|
| C₁₈H₃₇OH | 2 moles |
| Méthylènedicyclohexyldiisocyanate | 4 moles |
| Polyéthylèneglycol | 1 mole |
| N-méthyléthanolamine | 2 moles |
| Agent quaternisant (CH₃)₂SO₄ | 2 moles |

Le polymère 2 est le polymère suivant :
C₁₈H₃₇N⁺(CH₃)(CH₃)-(CH₂)₂-O-CONHR₄NHCO-O(POE)O-CONHR₄NHCO-O(CH₂)₂-N⁺(CH₃)(CH₃)C₁₈H₃₇
avec :
R₄ = méthylènedicyclohexyle
Contre ion : Cl⁻

Il est synthétisé à partir des réactifs suivants :

| | |
|---|---|
| Méthylènedicyclohexyldiisocyanate | 2 moles |
| Polyéthylèneglycol | 1 mole |
| N,N-diméthyléthanolamine | 2 moles |
| Agent quaternisant C₁₈H₃₇OH | 2 moles |

Les compositions de décoloration ci-dessus ont permis une décoloration régulière des cheveux teints artificiellement par une teinture d'oxydation.

### EXEMPLE 2 :

On a préparé les compositions de déformation permanente suivantes (exprimées en grammes):

| | C | D |
|---|---|---|
| Acide thioglycolique | 9,2 | 9,2 |
| Ammoniaque à 20% de NH3 | 9,3 | 9,3 |
| Carbonate d'ammonium | 4,5 | 4,5 |
| Cocoylamidopropylbétaïne / Monolaurate de glycérol (25/5) | 0,4MA* | 0,4 MA* |
| EDTA | 0,4 | 0,4 |
| Polymère cationique de formule W en solution à 60% dans l'eau | 1 MA* | 1MA* |
| Polymère 1 | 0,3 MA* | |
| Polymère 2 | | 0,3MA* |
| Eau qsp | 100 | 100 |

| | | |
|---|---|---|
| MA* = Matière Active | | |

Les compositions de déformation permanente ci-dessus ont été appliquées 15 minutes sur des cheveux mouillés préalablement enroulés sur des rouleaux de mise en plis, puis rincées abondamment à l'eau. On a appliqué alors pendant 5 minutes une solution d'eau oxygénée à 8 volumes et de pH 3, puis on a rincé à nouveau, on a enlevé les rouleaux et on a séché.
Les cheveux ont présenté dans les deux cas une belle frisure homogène.

## Revendications

1. Composition prête à l'emploi pour la décoloration ou la déformation des fibres kératiniques comprenant, dans un milieu approprié pour la décoloration ou la déformation permanente, au moins un agen réducteur, **caractérisée par le fait qu'**elle comprend en outre au moins un polyuréthane associatif cationique de formule (la) suivante :
R-X-(P)ₙ-[L-(Y)ₘ]ᵣ-L'-(P')ₚ-X'-R' (la)
dans laquelle:
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L'et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ;
la molécule contenant au moins une fonction amine protonée ou quatemisée et au moins un groupement hydrophobe.

2. Composition selon la revendication 1 **caractérisée par** le fait R et R' représentent tous les deux indépendamment un groupement hydrophobe, X, X' représentent chacun un groupe L", n et p valent entre 1 1 et 1000 et L, L', L", P, P', Y et m ont la signification indiquée dans la revendication 1.

3. Composition selon la revendication 1, **caractérisée par le fait que** R et R' représentent tous les deux indépendamment un groupement hydrophobe, X et X' représentent tous les deux Indépendamment un groupement comportant une amine quaternaire, n et p valent zéro, et L, L', Y et m ont la signification indiquée dans la revendication 1.

4. Composition selon l'une quelconque des revendications 1 à 3, **caractérisée par le fait que** R et R' représentent un radical ou un polymère à chaîne hydrocarbonée, saturée ou non, linéaire ou ramifiée, dans laquelle un ou plusieurs des atomes de carbone peut être remplacé par un hétéroatome choisi parmi S, N, O et P. ou à chaîne siliconée ou perfluorée.

5. Composition selon l'une quelconque des revendications 1 à 4, **caractérisée par le fait que** X et X' représentent l'une des formules : 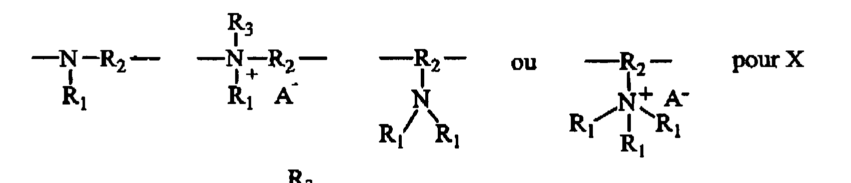 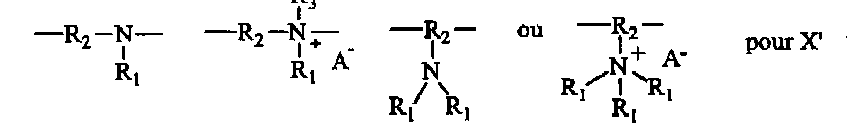 dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A⁻ est un contre-ion physiologiquement acceptable.

6. Composition selon l'une quelconque des revendications 1 à 5, **caractérisée par le fait que** les groupements L, L'et L", identiques ou différents, représentent la formule : 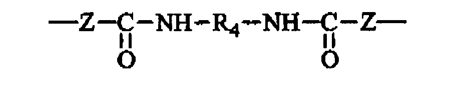 dans laquelle :
Z représente -O-, -S- ou -NH-; et
R₄ représente un radical alkylène ayant de 1 à 20 atomes de carbone, linéaire ou ramifié, comportant ou non un cycle saturé ou non saturé, un radical arylène, un ou plusieurs des atomes de carbone pouvant être remplacé par un hétéroatome choisi parmi N, S, O et P.

7. Composition selon l'une quelconque des revendications 1 à 6, **caractérisée par le fait que** les groupements P et P', identiques ou différents, représentent au moins l'une des formules suivantes : 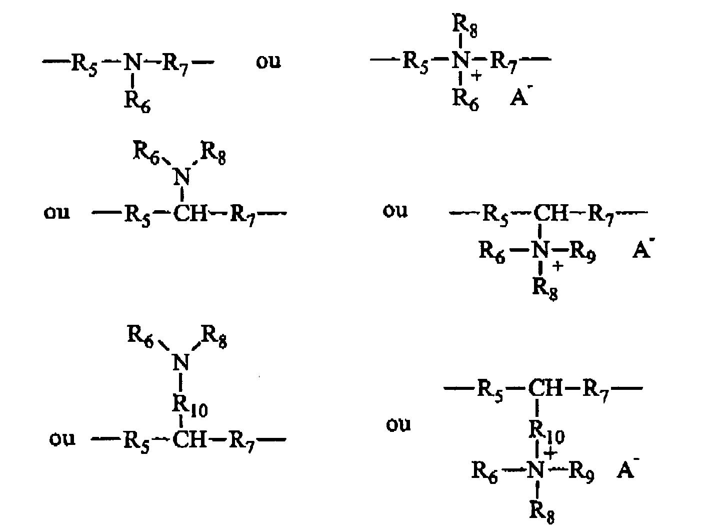 dans lesquelles :
R₅ et R₇ ont les mêmes significations que R₂;
R₆, R₈ et R₉ ont les mêmes significations que R₁ et R₃;
R₁₀ représente un groupe alkylène, linéaire ou ramifié, éventuellement insaturé et pouvant contenir un ou plusieurs hétéroatomes choisis parmi N, O, S et P, et
A⁻ est un contre-ion physiologiquement acceptable.

8. Composition selon l'une quelconque des revendications 1 à 7, **caractérisée par le fait que** Y représente un groupe dérivé d'éthylèneglycol, de diéthylèneglycol ou de propylèneglycol, ou un groupe dérivé d'un polymère choisi parmi les polyéthers, les polyesters sulfonés et les polyamides sulfonés.

9. Composition selon l'une quelconque des revendications précédentes, **caractérisée par le fait que** les polyuréthanes associatifs cationiques ont une masse moléculaire moyenne en nombre comprise entre 400 et 500000.

10. Composition selon l'une quelconque des revendications précédentes, **caractérisée par le fait que** les polyuréthanes associatifs cationiques sont utilisés en une quantité variant de 0,01 à 10% en poids du poids total de la composition.

11. Composition selon la revendication 10, **caractérisée par le fait que** les polyuréthanes associatifs cationiques sont utilisés en une quantité variant de 0.1 à 5% en poids du poids total de la composition.

12. Composition selon l'une quelconque des revendications précédentes, **caractérisée par le fait que** le ou les agents réducteurs sont choisis parmi les thiols tels que la cystéine, l'acide thioglycolique, l'acide thiolactique, leurs sels et leurs esters, la cystéamine et ses sels, ou les sulfites.

13. Composition selon l'une quelconque des revendications 1 à 11, **caractérisée par le fait que** les agents réducteurs sont choisis parmi l'acide ascorbique, ses sels et ses esters, l'acide érythorbique, ses sels et ses esters.

14. Composition selon l'une quelconque des revendications 12 ou 13, **caractérisée par le fait que** la concentration en agent réducteur peut varier de 0,1 à 30 % en poids par rapport au poids total de la composition.

15. Composition selon la revendication 14, **caractérisée par le fait qu'**elle peut varier de 0,5 à 20% en poids par rapport au poids total de la composition.

16. Composition selon l'une quelconque des revendications précédentes, **caractérisée par le fait qu'**elle comprend en outre au moins un polymère amphotère ou cationique différent des polyuréthanes associatifs cationiques tels que décrits à l'une quelconque des revendications 1 à 11.

17. Composition selon la revendication 16, **caractérisée par le fait que** le polymère cationique est un polymère de polyammonium quaternaire constitué de motifs récurrents répondant à la formule (W) suivante :

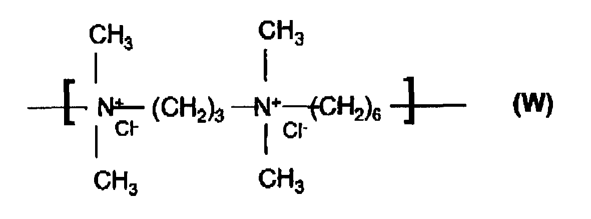

18. Composition selon la revendication 16, **caractérisée par le fait que** le polymère cationique est un polymère de polyammonium quaternaire constitué de motifs récurrents répondant à la formule (U) suivante :

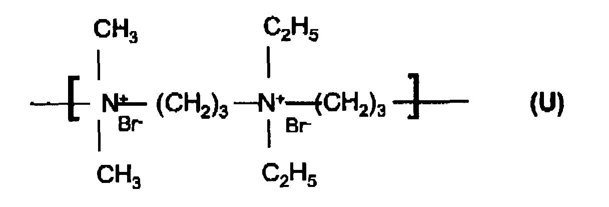

19. Composition selon la revendication 16, **caractérisée par le fait que** le polymère amphotère est un copolymère comprenant au moins comme monomères de l'acide acrylique et un sel de diméthyldiallylammonium.

20. Composition selon l'une quelconque des revendications 16 à 19, **caractérisée par le fait que** le ou les polymères cationiques ou amphotères représentent de 0,01 % à 10 %, en poids, du poids total de la composition.

21. Composition selon l'une quelconque des revendications précédentes, **caractérisée par le fait qu'**elle comprend au moins un tensioactif choisi parmi les tensioactifs anioniques, cationiques, non ioniques ou amphotères.

22. Composition selon la revendication 21, **caractérisée par le fait que** les tensioactifs représentent 0,01 à 40% en poids, du poids total de la composition.

23. Composition selon l'une quelconque des revendications précédentes, **caractérisée par le fait qu'**elle comprend au moins un agent épaississant additionnel.

24. Composition selon la revendication 23, **caractérisée par le fait que** l'agent épaississant additionnel est un dérivé de cellulose, un dérivé de guar, une gomme d'origine microbienne, un épaississant synthétique.

25. Composition selon les revendications 23-24, **caractérisée par le fait que** le ou les agents épaississants additionnels représentent 0,05 à 10% en poids du poids total de la composition.

26. Composition selon l'une quelconque des revendications précédentes, **caractérisée par le fait qu'**elle comprend en outre au moins un agent alcalinisant ou acidifiant dans des quantités allant de 0,01 à 30% en poids du poids total de la composition.

27. Composition selon la revendication 26, **caractérisée par le fait que** l'agent alcalinisant est choisi parmi l'ammoniaque, les carbonates alcalins, les alcanolamines telles que les mono-, di- et triéthanolamines ainsi que leurs dérivés, les hydroxyalkylamines et les éthylènediamines oxyéthylénées et/ou oxypropylénées, les hydroxydes de sodium ou de potassium et les composés de formule (XIX) suivante : 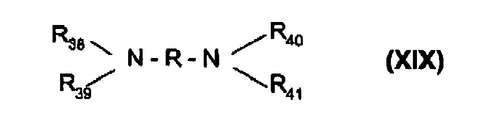 dans laquelle R est un reste propylène éventuellement substitué par un groupement hydroxyle ou un radical alkyle en C₁-C₄ ; R₃₈, R₃₉, R₄₀ et R₄₁, identiques ou différents, représentent un atome d'hydrogène, un radical alkyle en C₁-C₄ ou hydroxyalkyle en C₁-C₄.

28. Composition selon la revendication 26, **caractérisée par le fait que** l'agent acidifiant est choisi parmi les acides minéraux ou organiques comme l'acide chlorhydrique, l'acide orthophosphorique, les acides carboxyliques comme l'acide tartrique, l'acide citrique, l'acide lactique ou les acides sulfoniques.

29. Composition selon l'une quelconque des revendications 1 à 28, **caractérisée par le fait qu'**elle est obtenue par mélange extemporané au moment de l'emploi d'une composition anhydre comprenant au moins un agent réducteur et d'au moins une composition aqueuse, l'une au moins des compositions anhydre ou aqueuse comprenant au moins un polyuréthane associatif cationique.

30. Composition anhydre pour la décoloration ou la déformation des fibres kératiniques, comprenant au moins un agent réducteur, **caractérisée par le fait qu'**elle comprend en outre au moins un polyuréthane associatif cationique tel que défini dans l'une quelconque des revendications 1-11.

31. Composition selon les revendications 29 ou 30, **caractérisée par le fait que** la composition anhydre est sous forme pulvérulente.

32. Composition selon les revendications 29, 30 ou 31, **caractérisée par le fait que** la composition anhydre comprend au moins un additif choisi parmi les charges minérales ou organiques telles que la silice ou les argiles, les liants tels que la vinylpyrrolidone, les huiles ou les cires, les polyalkylèneglycols ou les dérivés de polyalkylèneglycols, les lubrifiants comme les stéarates de polyol ou les stéarates de métaux alcalins ou alcalino-terreux et les agents colorants ou matifiants comme les oxydes de titane.

33. Composition selon la revendication 32, **caractérisée par le fait que** chacun des additifs est présent à une concentration allant de 0 à 30% en poids du poids total de la composition.

34. Composition selon l'une quelconque des revendications 1 à 28, **caractérisée par le fait qu'**elle est aqueuse.

35. Composition selon la revendication 34, **caractérisée par le fait que** le milieu aqueux contient un ou plusieurs solvants organiques.

36. Composition selon la revendication 35, **caractérisée par le fait que** la concentration en solvant(s) varie de 0,5 à 20% en poids par rapport au poids total de la composition.

37. Composition selon l'une quelconque des revendications 1-29 et 31-36, **caractérisée par le fait que** la composition possède un pH allant de 1,5 à 12.

38. Composition pour la décoloration selon l'une quelconque des revendications 1-29 et 31-37, **caractérisée par le fait que** le pH est compris entre 1,5 et 10.

39. Composition pour la déformation permanente selon l'une quelconque des revendications 1-29 et 31-37, **caractérisée par le fait que** le pH est compris entre 6 et 12.

40. Composition selon la revendication 29, **caractérisée par le fait que** le polymère polyuréthane associatif cationique se trouve dans une composition aqueuse.

41. Procédé de décoloration ou de déformation permanente des fibres kératiniques, consistant à appliquer sur les fibres, sèches ou humides, une composition prête à l'emploi comprenant, dans un milieu approprié pour la décoloration, au moins un agent réducteur et au moins un polyuréthane associatif cationique tel que défini dans l'une quelconque des revendications 1 à 11, et à la laisser agir pendant un temps de pause variant de 1 à 60 minutes, et de préférence de 10 à 45 minutes, à rincer éventuellement les fibres, à les laver au shampooing, puis à les rincer à nouveau, et à les sécher, l'application de ladite composition pouvant être suivie, dans le cas de la déformation permanente, par l'application, éventuellement après rinçage, d'une composition oxydante qu'on laisse agir pendant un temps de pause compris entre 1 et 20 minutes et de préférence entre 1 et 10 minutes, puis on lave éventuellement au shampooing, on rince à nouveau, et on séche.

42. Dispositif à deux compartiments ou « Kit » pour la décoloration ou la déformation permanente des fibres kératiniques humaine, **caractérisé par le fait que** le premier compartiment comprend au moins une poudre ou une composition aqueuse, et le deuxième compartiment une composition aqueuse, l'un au moins des deux compartiments comprenant au moins un agent réducteur et l'un au moins des deux compartiments comprenant au moins un polyuréthane associatif cationique tel que défini à l'une quelconque des revendications 1-11.

## Claims

1. Ready-to-use composition for bleaching or reshaping keratin fibres, comprising, in a medium that is suitable for bleaching or permanent reshaping, at least one reducing agent, **characterized in that** it also comprises at least one cationic associative polyurethane of formula (Ia) below:
R-X-(P)ₙ-[L-(Y)ₘ]ᵣ-L'-(P')ₚ-X'-R' (Ia)
in which:
R and R', which may be identical or different, represent a hydrophobic group or a hydrogen atom;
X and X', which may be identical or different, represent a group comprising an amine function optionally bearing a hydrophobic group, or alternatively the group L";
L, L' and L", which may be identical or different, represent a group derived from a diisocyanate;
P and P', which may be identical or different, represent a group comprising an amine function optionally bearing a hydrophobic group;
Y represents a hydrophilic group;
r is an integer between 1 and 100, preferably between 1 and 50 and in particular between 1 and 25,
n, m and p each range, independently of each other, from 0 to 1000;
the molecule containing at least one protonated or quaternized amine function and at least one hydrophobic group.

2. Composition according to Claim 1, **characterized in that** R and R' both independently represent a hydrophobic group, X and X' each represent a group L", n and p are between 1 and 1000, and L, L', L", P, P', Y and m have the meaning given in Claim 1.

3. Composition according to Claim 1, **characterized in that** R and R' both independently represent a hydrophobic group, X and X' both independently represent a group comprising a quaternary amine, n and p are zero, and L, L', Y and m have the meaning given in Claim 1.

4. Composition according to any one of Claims 1 to 3, **characterized in that** R and R' represent a radical or polymer containing a saturated or unsaturated, linear or branched hydrocarbon-based chain, in which one or more of the carbon atoms may be replaced with a hetero atom chosen from S, N, O and P, or a radical or polymer containing a perfluoro or silicone chain.

5. Composition according to any one of Claims 1 to 4, **characterized in that** X and X' represent one of the formulae: 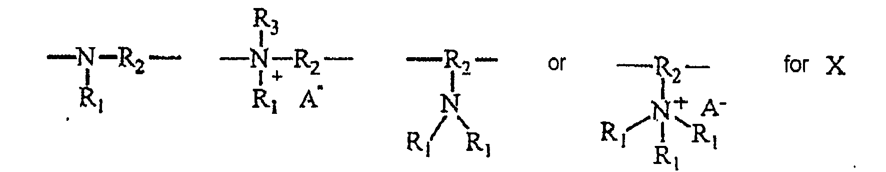 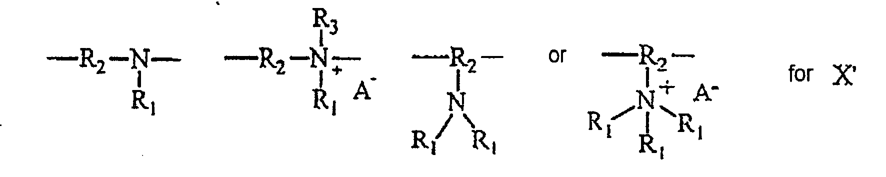 in which:
R₂ represents a linear or branched alkylene radical containing from 1 to 20 carbon atoms, optionally comprising a saturated or unsaturated ring, or an arylene radical, one or more of the carbon atoms possibly being replaced with a hetero atom chosen from N, S, O and P;
R₁ and R₃, which may be identical or different, denote a linear or branched C₁-C₃₀ alkyl or alkenyl radical, or an aryl radical, at least one of the carbon atoms possibly being replaced with a hetero atom chosen from N, S, O and P;
A⁻ is a physiologically acceptable counterion.

6. Composition according to any one of Claims 1 to 5, **characterized in that** the groups L, L' and L", which may be identical or different, represent the formula: 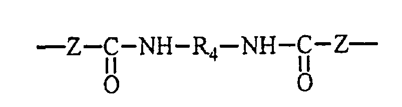 in which:
Z represents -O-, -S- or -NH-; and
R₄ represents a linear or branched alkylene radical containing from 1 to 20 carbon atoms, optionally comprising a saturated or unsaturated ring, or an arylene radical, one or more of the carbon atoms possibly being replaced with a hetero atom chosen from N, S, O and P.

7. Composition according to any one of Claims 1 to 6, **characterized in that** the groups P and P', which may be identical or different, represent at least one of the following formulae: 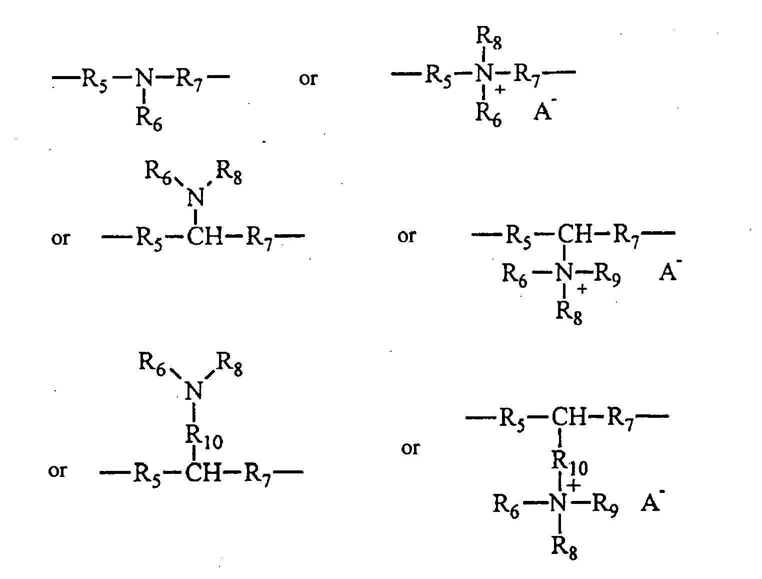 in which:
R₅ and R₇ have the same meanings as R₂;
R₆, R₈ and R₉ have the same meanings as R₁ and R₃;
R₁₀ represents a linear or branched, optionally unsaturated alkylene group possibly containing one or more hetero atoms chosen from N, O, S and P; and
A⁻ is a physiologically acceptable counterion.

8. Composition according to any one of Claims 1 to 7, **characterized in that** Y represents a group derived from ethylene glycol, from diethylene glycol or from propylene glycol, or a group derived from a polymer chosen from polyethers, sulfonated polyesters and sulfonated polyamides.

9. Composition according to any one of the preceding claims, **characterized in that** the cationic associative polyurethanes have a number-average molecular mass of between 400 and 500 000.

10. Composition according to any one of the preceding claims, **characterized in that** the cationic associative polyurethanes are used in an amount ranging from 0.01% to 10% by weight relative to the total weight of the composition.

11. Composition according to Claim 10, **characterized in that** the cationic associative polyurethanes are used in an amount ranging from 0.1% to 5% by weight relative to the total weight of the composition.

12. Composition according to any one of the preceding claims, **characterized in that** the reducing agent(s) is (are) chosen from thiols such as cysteine, thioglycolic acid, thiolactic acid, salts thereof and esters thereof, cysteamine and its salts, and sulfites.

13. Composition according to any one of Claims 1 to 11, **characterized in that** the reducing agent(s) is (are) chosen from ascorbic acid, salts thereof and esters thereof, and erythorbic acid, salts thereof and esters thereof.

14. Composition according to either of Claims 12 and 13, **characterized in that** the concentration of reducing agent may range from 0.1% to 30% by weight relative to the total weight of the composition.

15. Composition according to Claim 14, **characterized in that** it can range from 0.5% to 20% by weight relative to the total weight of the composition.

16. Composition according to any one of the preceding claims, **characterized in that** it also comprises at least one amphoteric or cationic polymer other than the cationic associative polyurethanes as described in any one of Claims 1 to 11.

17. Composition according to Claim 16, **characterized in that** the cationic polymer is a polyquaternary ammonium polymer consisting of repeating units corresponding to formula (W) below:

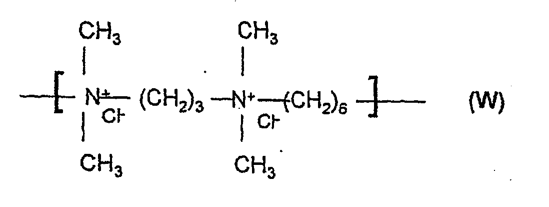

18. Composition according to Claim 16, **characterized in that** the cationic polymer is a polyquaternary ammonium polymer consisting of repeating units corresponding to formula (U) below:

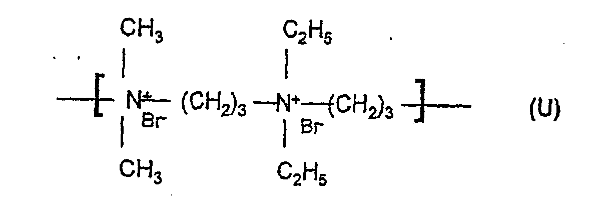

19. Composition according to Claim 16, **characterized in that** the amphoteric polymer is a copolymer comprising at least, as monomers, acrylic acid and a dimethyldiallylammonium salt.

20. Composition according to any one of Claims 16 to 19, **characterized in that** the cationic or amphoteric polymer(s) represent(s) from 0.01% to 10% by weight, relative to the total weight of the composition.

21. Composition according to any one of the preceding claims, **characterized in that** it comprises at least one surfactant chosen from anionic, cationic, nonionic and amphoteric surfactants.

22. Composition according to Claim 21, **characterized in that** the surfactants represent from 0.01% to 40% by weight, relative to the total weight of the composition.

23. Composition according to any one of the preceding claims, **characterized in that** it comprises at least one additional thickener.

24. Composition according to Claim 23, **characterized in that** the additional thickener is a cellulose derivative, a guar derivative, a gum of microbial origin or a synthetic thickener.

25. Composition according to Claims 23-24, **characterized in that** the additional thickener(s) represent(s) from 0.05% to 10% by weight, relative to the total weight of the composition.

26. Composition according to any one of the preceding claims, **characterized in that** it also comprises at least one basifying or acidifying agent in amounts ranging from 0.01% to 30% by weight relative to the total weight of the composition.

27. Composition according to Claim 26, **characterized in that** the basifying agent is chosen from aqueous ammonia, alkali metal carbonates, alkanolamines such as monoethanolamine, diethanolamine and triethanolamine, and derivatives thereof, oxyethylenated and/or oxypropylenated hydroxyalkylamines and ethylenediamines, sodium hydroxide, potassium hydroxide and the compounds of formula (XIX) below: 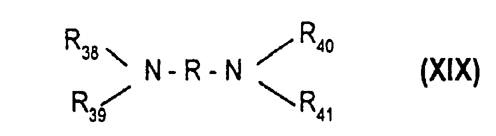 in which R is a propylene residue optionally substituted with a hydroxyl group or a C₁-C₄ alkyl radical; R₃₈, R₃₉, R₄₀ and R₄₁, which may be identical or different, represent a hydrogen atom or a C₁-C₄ alkyl or C₁-C₄ hydroxyalkyl radical.

28. Composition according to Claim 26, **characterized in that** the acidifying agent is chosen from mineral or organic acids, for instance hydrochloric acid, orthophosphoric acid, carboxylic acids, for instance tartaric acid, citric acid or lactic acid, and sulfonic acids.

29. Composition according to any one of Claims 1 to 28, **characterized in that** it is obtained by extemporaneous mixing at the time of use of an anhydrous composition comprising at least one reducing agent and at least one aqueous composition, at least one of the anhydrous or aqueous compositions comprising at least one cationic associative polyurethane.

30. Anhydrous composition for bleaching or reshaping keratin fibres, comprising at least one reducing agent, **characterized in that** it also comprises at least one cationic associative polyurethane as defined in any one of Claims 1-11.

31. Composition according to Claim 29 or 30, **characterized in that** the anhydrous composition is in pulverulent form.

32. Composition according to Claim 29, 30 or 31, **characterized in that** the anhydrous composition comprises at least one additive chosen from mineral or organic fillers such as silica or clays, binders such as vinylpyrrolidone, oils or waxes, polyalkylene glycols or polyalkylene glycol derivatives, lubricants, for instance polyol stearates or alkali metal or alkaline-earth metal stearates, and colorants or matting agents, for instance titanium oxides.

33. Composition according to Claim 32, **characterized in that** each of the additives is present at a concentration ranging from 0 to 30% by weight relative to the total weight of the composition.

34. Composition according to any one of Claims 1 to 28, **characterized in that** it is aqueous.

35. Composition according to Claim 34, **characterized in that** the aqueous medium contains one or more organic solvents.

36. Composition according to Claim 35, **characterized in that** the concentration of solvent(s) ranges from 0.5% to 20% by weight relative to the total weight of the composition.

37. Composition according to any one of Claims 1-29 and 31-36, **characterized in that** the composition has a pH ranging from 1.5 to 12.

38. Bleaching composition according to any one of Claims 1-29 and 31-37, **characterized in that** the pH is between 1.5 and 10.

39. Permanent-reshaping composition according to any one of Claims 1-29 and 31-37, **characterized in that** the pH is between 6 and 12.

40. Composition according to Claim 29, **characterized in that** the cationic associative polyurethane polymer is in an aqueous composition.

41. Process for bleaching or permanently reshaping keratin fibres, which consists in applying to the wet or dry fibres a ready-to-use composition comprising, in a medium that is suitable for bleaching, at least one reducing agent and at least one cationic associative polyurethane as defined in any one of Claims 1 to 11, and in leaving the composition to act for an action time ranging from 1 to 60 minutes and preferably from 10 to 45 minutes, optionally rinsing the fibres, washing them with shampoo, followed by rinsing them again and drying them, the application of said composition possibly being followed, in the case of permanent reshaping, by the application, optionally after rinsing, of an oxidizing composition that is left to act for an action time of between 1 and 20 minutes and preferably between 1 and 10 minutes, optionally followed by washing with shampoo, rinsing again and drying.

42. Two-compartment device or kit for bleaching or permanently reshaping human keratin fibres, **characterized in that** the first compartment comprises at least one powder or one aqueous composition, and the second compartment comprises an aqueous composition, at least one of the two compartments comprising at least one reducing agent and at least one of the two compartments comprising at least one cationic associative polyurethane as defined in any one of Claims 1-11.

## Patentansprüche

1. Gebrauchsfertige Zusammensetzung für die Entfärbung oder dauerhafte Verformung von Keratinfasern, die in einem zum Entfärben oder dauerhaften Verformen geeigneten Medium mindestens ein Reduktionsmittel enthält, **dadurch gekennzeichnet, dass** sie ferner mindestens ein kationisches assoziatives Polyurethan der folgenden Formel (Ia) enthält:
R-X-(P)ₙ-[L-(Y)ₘ]ᵣ-L'-(P')ₚ-X'-R' (Ia),
worin bedeuten:
R und R', die gleich oder verschieden sind, eine hydrophobe Gruppe oder ein Wasserstoffatom,
X und X', die gleich oder verschieden sind, eine Gruppe, die eine Aminofunktion enthält, die gegebenenfalls eine hydrophobe Gruppe aufweist, oder die Gruppe L",
L, L' oder L", die gleich oder verschieden sind, eine Gruppe, die von einem Diisocyanat abgeleitet ist,
P und P', die gleich oder verschieden sind, eine Gruppe, die eine Aminofunktion enthält, die gegebenenfalls eine hydrophobe Gruppe aufweist,
Y eine hydrophile Gruppe,
r eine ganze Zahl von 1 bis 100 , vorzugsweise 1 bis 50 und insbesondere 1 bis 25,
n, m und p unabhängig voneinander Werte von 0 bis 1000,
wobei das Molekül mindestens eine protonierte oder quaternisierte Aminofunktion und mindestens eine hydrophobe Gruppe enthält.

2. Zusammensetzung nach Anspruch 1, **dadurch gekennzeichnet, dass** R und R' beide unabhängig voneinander eine hydrophobe Gruppe, X und X' jeweils L" und n und p Werte von 1 bis 1000 bedeuten und L, L', L", P, P', Y und m die in Anspruch 1 angegebenen Bedeutungen aufweisen.

3. Zusammensetzung nach Anspruch 1, **dadurch gekennzeichnet, dass** R und R' beide unabhängig voneinander eine hydrophobe Gruppe, X und X' beide unabhängig voneinander eine Gruppe, die eine quartäre Aminogruppe enthält, und n und p Null bedeuten und L, L', Y und m die in Anspruch 1 angegebenen Bedeutungen aufweisen.

4. Zusammensetzung nach einem der Ansprüche 1 bis 3, **dadurch gekennzeichnet, dass** R und R' eine Gruppe oder ein Polymer mit einer geradkettigen oder verzweigten, gesättigten oder ungesättigten Kohlenwasserstoffkette, in der ein oder mehrere Kohlenstoffatome durch ein Heteroatom ersetzt sein können, das unter S, N, O oder P ausgewählt ist, oder mit einer siliconierten Kette oder perfluorierten Kette bedeuten.

5. Zusammensetzung nach einem der Ansprüche 1 bis 4, **dadurch gekennzeichnet, dass** X und X' durch eine der folgenden Formeln dargestellt werden können: 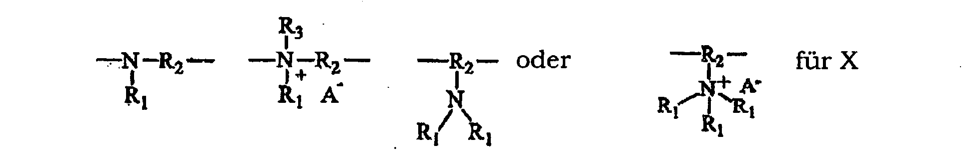 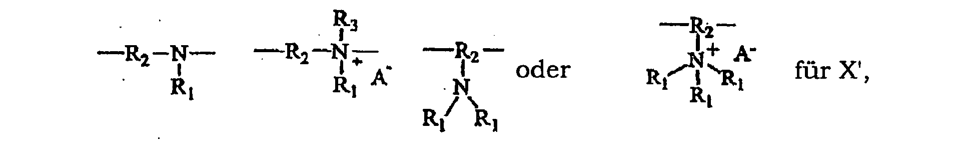 worin bedeuten:
R₂ eine geradkettige oder verzweigte Alkylengruppe mit 1 bis 20 Kohlenstoffatomen, die gegebenenfalls einen gesättigten oder ungesättigten Ring umfasst, oder eine Arylengruppe, wobei ein oder mehrere Kohlenstoffatome durch ein Heteroatom ersetzt sein können, das unter N, S, O und P ausgewählt ist;
R₁ und R₃, die gleich oder verschieden sind, eine geradkettige oder verzweigte Alkyl- oder Alkenylgruppe mit 1 bis 30 Kohlenstoffatomen, eine Arylgruppe, wobei mindestens ein Kohlenstoffatom durch ein Heteroatom ersetzt sein kann, das unter N, S, O und P ausgewählt ist; und
A⁻ ein physiologisch akzeptables Gegenion.

6. Zusammensetzung nach einem der Ansprüche 1 bis 5, **dadurch gekennzeichnet, dass** die Gruppen L, L' und L", die gleich oder verschieden sind, eine Gruppe der folgenden Formel bedeuten: 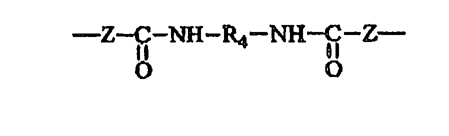 worin bedeuten:
Z -O-, -S- oder -NH-; und
R₄ eine geradkettige oder verzweigte Alkylengruppe mit 1 bis 20 Kohlenstoffatomen, die gegebenenfalls einen gesättigten oder ungesättigten Ring umfasst, oder eine Arylengruppe, wobei ein oder mehrere Kohlenstoffatome durch ein Heteroatom ersetzt sein können, das unter N, S, O und P ausgewählt ist.

7. Zusammensetzung nach einem der Ansprüche 1 bis 6, **dadurch gekennzeichnet, dass** die Gruppen P und P', die gleich oder verschieden sind, durch mindestens eine der folgenden Formeln dargestellt werden können: 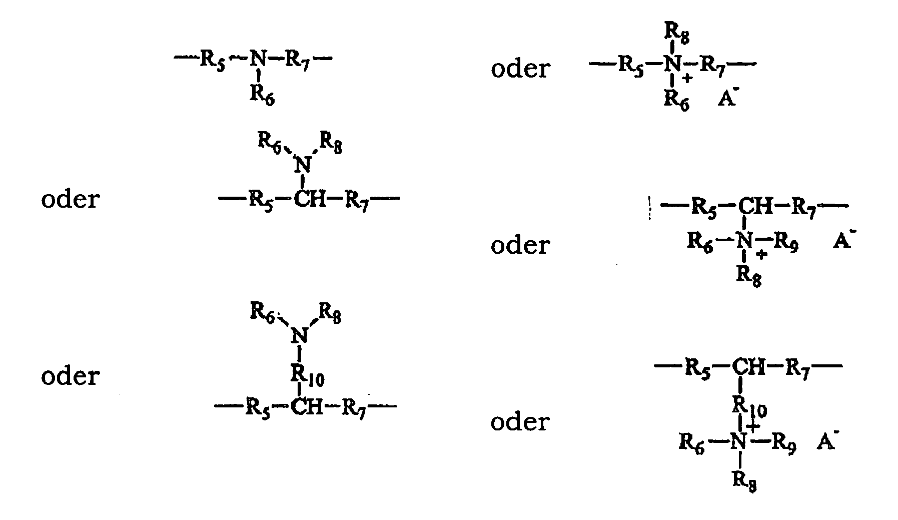 worin bedeuten:
R₅ und R₇ die für R₂ angegebenen Bedeutungen,
R₆, R₈ und R₉ die für R₁ und R₃ angegebenen Bedeutungen,
Rio eine geradkettige oder verzweigte, gegebenenfalls ungesättigte Alkylengruppe, die ein oder mehrere Heteroatome enthalten kann, die unter N, O, S und P ausgewählt sind; und
A- ein physiologisch akzeptables Gegenion.

8. Zusammensetzung nach einem der Ansprüche 1 bis 7, **dadurch gekennzeichnet, dass** Y eine Gruppe, die von Ethylenglycol, Diethylenglycol und Propylenglycol abgeleitet ist, oder eine Gruppe bedeutet, die von einem Polymer stammt, das unter den Polyethern, sulfonierten Polyestern und sulfonierten Polyamiden ausgewählt ist.

9. Zusammensetzung nach einem der vorhergehenden Ansprüche, **dadurch gekennzeichnet, dass** die kationischen assoziativen Polyurethane eine zahlenmittlere Molmasse von 400 bis 500 000 besitzen.

10. Zusammensetzung nach einem der vorhergehenden Ansprüche, **dadurch gekennzeichnet, dass** die kationischen assoziativen Polyurethane in einer Menge von 0,01 bis 10 Gew.-% des Gesamtgewichts der Zusammensetzung verwendet werden.

11. Zusammensetzung nach Anspruch 10, **dadurch gekennzeichnet, dass** die kationischen assoziativen Polyurethane in einer Menge von 0,1 bis 5 Gew.-% des Gesamtgewichts der Zusammensetzung verwendet werden.

12. Zusammensetzung nach einem der vorhergehenden Ansprüche, **dadurch gekennzeichnet, dass** das oder die Reduktionsmittel unter Thiolen, wie Cystein, Thioglycolsäure, Thiomilchsäure, ihren Salzen und ihren Estern, Cysteamin und seinen Salzen, oder Sulfiten ausgewählt sind.

13. Zusammensetzung nach einem der Ansprüche 1 bis 11, **dadurch gekennzeichnet, dass** die Reduktionsmittel unter Ascorbinsäure, ihren Salzen und ihren Estern, Erythorbinsäure, ihren Salzen und ihren Estern ausgewählt ist.

14. Zusammensetzung nach einem der Ansprüche 12 oder 13, **dadurch gekennzeichnet, dass** die Konzentration des Reduktionsmittels im Bereich von 0,1 bis 30 Gew.-%, bezogen auf das Gesamtgewicht der Zusammensetzung, liegen kann.

15. Zusammensetzung nach Anspruch 14, **dadurch gekennzeichnet, dass** die Konzentration im Bereich von 0,5 bis 20 Gew.-% liegen kann.

16. Zusammensetzung nach einem der vorhergehenden Ansprüche, **dadurch gekennzeichnet, dass** sie ferner mindestens ein kationisches oder amphoteres Polymer enthält, das von den in den Ansprüchen 1 bis 11 beschriebenen kationischen assoziativen Polyurethanen verschieden ist.

17. Zusammensetzung nach Anspruch 16, **dadurch gekennzeichnet, dass** das kationische Polymer ein quartäres Polyammoniumpolymer ist, das aus Wiederholungseinheiten der folgenden Formel (W) besteht:

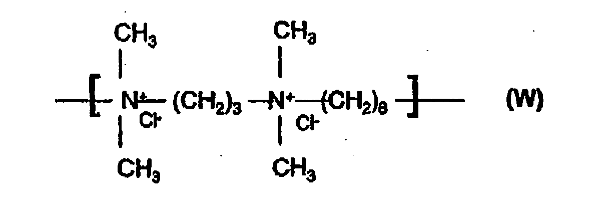

18. Zusammensetzung nach Anspruch 16, **dadurch gekennzeichnet, dass** das kationische Polymer ein quartäres Polyammoniumpolymer ist, das aus Wiederholungseinheiten der folgenden Formel (U) besteht:

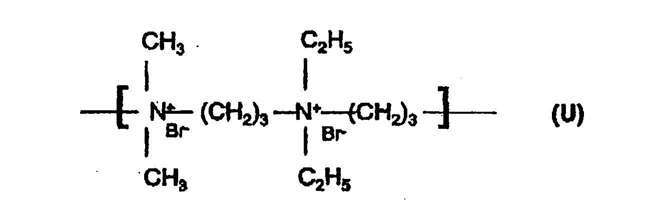

19. Zusammensetzung nach Anspruch 16, **dadurch gekennzeichnet, dass** das amphotere Polymer ein Copolymer ist, das als Monomere zumindest Acrylsäure und ein Dimethyldiallylammoniumsalz enthält.

20. Zusammensetzung nach einem der Ansprüche 16 bis 19, **dadurch gekennzeichnet, dass** das oder die kationische(n) oder amphotere(n) Polymer(e) 0,01 bis 10 Gew.-% des Gesamtgewichts der Zusammensetzung ausmachen.

21. Zusammensetzung nach einem der vorhergehenden Ansprüche, **dadurch gekennzeichnet, dass** sie mindestens einen grenzflächenaktiven Stoff enthält, der unter den anionischen, kationischen, nichtionischen oder amphoteren grenzflächenaktiven Stoffen ausgewählt ist.

22. Zusammensetzung nach Anspruch 21, **dadurch gekennzeichnet, dass** die grenzflächenaktiven Stoffe 0,01 bis 40 Gew.-% des Gesamtgewichts der Zusammensetzung ausmachen.

23. Zusammensetzung nach einem der vorhergehenden Ansprüche, **dadurch gekennzeichnet, dass** sie mindestens ein zusätzliches Verdickungsmittel enthält.

24. Zusammensetzung nach Anspruch 23, **dadurch gekennzeichnet, dass** das zusätzliche Verdickungsmittel ein Cellulosederivat, Guarderivat, Gummi mikrobieller Herkunft oder synthetisches Verdickungsmittel ist.

25. Zusammensetzung nach den Ansprüchen 23 bis 24, **dadurch gekennzeichnet, dass** das oder die ergänzende(n) Verdickungsmittel 0,05 bis 10 Gew.-% des Gesamtgewichts der Zusammensetzung ausmachen.

26. Zusammensetzung nach einem der vorhergehenden Ansprüche, **dadurch gekennzeichnet, dass** sie ferner mindestens ein Alkalisierungsmittel oder Ansäuerungsmittel in Mengenanteilen von 0,01 bis 30 % des Gesamtgewichts der Zusammensetzung enthält.

27. Zusammensetzung nach Anspruch 26, **dadurch gekennzeichnet, dass** das Alkalisierungsmittel unter Ammoniak, , Alkalicarbonaten, Alkanolaminen, wie Mono-, Di- und Triethanolamin, sowie deren Derivaten, Hydroxyalkylaminen und ethoxylierten und/ oder propoxylierten Ethylendiaminen, Natriumhydroxid oder Kaliumhydroxid und den Verbindungen der folgenden Formel (XIX) ausgewählt ist: 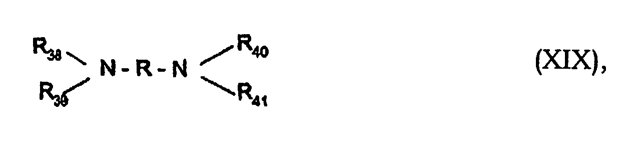 worin R eine gegebenenfalls mit einer Hydroxygruppe oder einer C₁₋₄-Alkylgruppe substituierte Propylengruppe ist; und die Gruppen R₃₈, R₃₉, R₄₀ und R₄₁, die gleich oder verschieden sind, Wasserstoff, C₁₋₄-Alkyl oder C₁₋₄-Hydroxyalkyl bedeuten.

28. Zusammensetzung nach Anspruch 26, **dadurch gekennzeichnet, dass** das Ansäuerungsmittel unter den anorganischen oder organischen Säuren ausgewählt ist, wie Salzsäure, Orthophosphorsäure, Carbonsäuren, wie Weinsäure, Citronensäure, Milchsäure, oder Sulfonsäuren.

29. Zusammensetzung nach einem der Ansprüche 1 bis 28, **dadurch gekennzeichnet, dass** sie durch bedarfsgemäßes Mischen einer wasserfreien Zusammensetzung, die mindestens ein Reduktionsmittel enthält, und mindestens einer wässrigen Zusammensetzung bei der Anwendung hergestellt wird, wobei mindestens eine der Zusammensetzungen, die wasserfreie oder die wässrige Zusammensetzung, mindestens ein kationisches assoziatives Polyurethan enthält.

30. Wasserfreie Zusammensetzung zum Entfärben oder dauerhaften Verformen von Keratinfasern, die mindestens ein Reduktionsmittel enthält, **dadurch gekennzeichnet, dass** sie ferner mindestens ein assoziatives kationisches Polyurethan enthält, wie es in einem der Ansprüche 1 bis 11 definiert wurde.

31. Zusammensetzung nach den Ansprüchen 29 oder 30, **dadurch gekennzeichnet, dass** die wasserfreie Zusammensetzung pulverförmig vorliegt.

32. Zusammensetzung nach den Ansprüchen 29, 30 oder 31, **dadurch gekennzeichnet, dass** die wasserfreie Zusammensetzung mindestens einen Zusatzstoff enthält, der unter den anorganischen oder organischen Füllstoffen, wie Kieselsäure oder Tonen, Bindemitteln, wie Vinylpyrrolidon, Ölen oder Wachsen, Polyalkylenglycolen oder Polyalkylenglycolderivaten, Gleitmitteln wie Polyolstearaten oder Alkali- oder Erdalkalistearaten, Farbmitteln oder Mattierungsmitteln wie Titanoxiden ausgewählt ist.

33. Zusammensetzung nach Anspruch 32, **dadurch gekennzeichnet, dass** jeder Zusatzstoff in einer Konzentration von 0 bis 30 Gew.-% des Gesamtgewichts der Zusammensetzung enthalten ist.

34. Zusammensetzung nach einem der Ansprüche 1 bis 28, **dadurch gekennzeichnet, dass** sie wässrig ist.

35. Zusammensetzung nach Anspruch 34, **dadurch gekennzeichnet, dass** das wässrige Medium ein oder mehrere organische Lösungsmittel enthält.

36. Zusammensetzung nach Anspruch 35, **dadurch gekennzeichnet, dass** die Konzentration des Lösungsmittels oder der Lösungsmittel im Bereich von 0,5 bis 20 Gew.-%, bezogen auf das Gesamtgewicht der Zusammensetzung, liegt.

37. Zusammensetzung nach einem der Ansprüche 1 bis 19 und 31 bis 36, **dadurch gekennzeichnet, dass** die Zusammensetzung einen pH-Wert von 1,5 bis 12 aufweist

38. Zusammensetzung zum Entfärben nach einem der Ansprüche 1 bis 29 und 31 bis 37, **dadurch gekennzeichnet, dass** der pH-Wert im Bereich von 1,5 bis 10 liegt.

39. Zusammensetzung zum dauerhaften Verformen nach einem der Ansprüche 1 bis 29 und 31 bis 37, **dadurch gekennzeichnet, dass** der pH-Wert im Bereich von 6 bis 12 liegt.

40. Zusammensetzung nach Anspruch 29, **dadurch gekennzeichnet, dass** das kationische assoziative Polyurethan in der wässrigen Zusammensetzung enthalten ist.

41. Verfahren zum Entfärben oder dauerhaften Verformen von Keratinfasern, das darin besteht, auf die trockenen oder feuchten Fasern eine gebrauchsfertige Zusammensetzung aufzutragen, die in einem zum Entfärben geeigneten Medium mindestens ein Reduktionsmittel und mindestens ein in Anspruch 1 bis 11 definiertes kationisches assoziatives Polyurethan enthält, und während einer Zeitspanne von 1 bis 60 min und vorzugsweise 10 bis 45 min einwirken zu lassen, die Fasern gegebenenfalls zu spülen, mit Haarwaschmittel zu waschen und nochmals zu spülen und die Fasern zu trocknen, wobei bei einer dauerhaften Verformung nach der Anwendung der Zusammensetzung gegebenenfalls nach einem Spülschritt eine oxidierende Zusammensetzung aufgetragen und während einer Zeitspanne von 1 bis 20 min und vorzugsweise 1 bis 10 min einwirken gelassen wird, wonach gegebenenfalls mit Haarwaschmittel gewaschen, nochmals gespült und getrocknet wird.

42. Vorrichtung mit zwei Abteilungen oder 'Kit' zum Entfärben oder dauerhaften Verformen von menschlichen Keratinfasern, **dadurch gekennzeichnet, dass** die erste Abteilung mindestens ein Pulver oder eine wässrige Zusammensetzung und die zweite Abteilung eine wässrige Zusammensetzung enthält, wobei mindestens eine der beiden Abteilungen mindestens ein Reduktionsmittel und mindestens eine der beiden Abteilungen mindestens ein in Anspruch 1 bis 11 definiertes kationisches assoziatives Polyurethan enthält.
